# DEMANDE DE BREVET EUROPEEN

(11) **EP 2 808 047 A1**
(43) Date de publication de la demande: **03.12.2014**
(21) Numéro de dépôt: 13169405.1
(22) Date de dépôt: 27.05.2013
(51) Int. Cl.: A61M 5/32

(54) **Dispositif de condamnation automatique, notamment pour une seringue et seringue comportant un tel dispositif**

(71) Demandeur: Biofront, 75014 Paris (FR)
(72) Inventeur: Lahlou, Pierre Khalid, 75014 PARIS (FR)
(74) Mandataire: Hamann, Jean-Christophe

(57) **Abrégé**

L'invention concerne un dispositif de verrouillage automatique d'une canule (140) d'injection, notamment pour une seringue, lequel dispositif comporte :
a. un bouclier (220) sensiblement tubulaire de longueur égale ou supérieur à celle de la canule à verrouiller ;
b. un moyen (210) de guidage apte à guider ledit bouclier (120) selon une translation axiale parallèle à l'axe (101) du cylindre ;
c. des moyens (230) élastiques, dits de rappel, aptes à exercer une pression axiale sur le bouclier (220) entre une première position, dite proximale ou d'armement, où ledit bouclier (220) découvre la canule et une deuxième position, dite distale ou de sécurité, où ledit bouclier (220) recouvre axialement la canule ;
d. des moyens (224, 214) de verrouillage, aptes à bloquer le bouclier dans la position distale ;
e. caractérisé en ce qu'il comprend des moyens (223, 213) d'arrêt aptes à arrêter le bouclier (220) dans une position axiale, dite intermédiaire ou pré-opératoire, entre la position distale et la position proximale, les moyens (230) élastiques de rappel exerçant une pression axiale orientée de la position proximale vers la position distale, par l'intermédiaire du bouclier (220), sur lesdits moyens d'arrêt; et
f. des moyens (222) pour escamoter les moyens d'arrêt lorsque le bouclier passe de la position proximale à la position distale sous l'effet de la détente des moyens élastiques (230)de rappel.

## Description

L'invention concerne un dispositif de condamnation automatique, notamment pour une seringue, et une seringue comportant un tel dispositif. L'invention est plus particulièrement, mais non exclusivement, adaptée à une seringue fabriquée et remplie en grande série par des moyens automatisés.

Selon un exemple de production automatisée, au cours d'une première étape, le corps de la seringue est fabriqué, par exemple sous la forme d'un tube en verre, comportant à une première extrémité longitudinale une interface apte à recevoir une canule et à l'extrémité opposée, une collerette dite de prise digitale. La première extrémité étant bouchée par un capuchon, ou par une canule coiffée d'un capuchon, une pluralité de corps de seringue est regroupée de manière matricielle sur un plateau à cheminées. Cet ensemble est stérilisé et emballé de manière hermétique, formant une boîte. Pour le remplissage, ladite boîte est placée dans une machine de remplissage automatique, située le cas échéant sur un site éloigné, où elle est ouverte automatiquement en environnement stérile. Les corps de seringue sont suspendus verticalement dans le plateau à cheminées par la collerette de prise digitale, de sorte à présenter ledit corps ouvert au dispositif de remplissage. Ledit dispositif de remplissage rempli les corps de seringue par une pluralité de canules, généralement rangée par rangée. Puis, les pistons sont installés sur lesdits corps de seringue par des moyens automatisés.

L'utilisation de tels moyens automatisés est indispensable pour la production de moyens thérapeutiques en masse et à bas coût, notamment des vaccins, par exemple pour le traitement d'une pandémie.

Compte tenu des possibilités de propagation d'agents infectieux par l'intermédiaires des seringues, soit par la réutilisation de celles-ci, soit par piqûre accidentelle, l'Organisation Mondiale de la Santé préconise l'utilisation de seringues de sécurité autobloquantes plus particulièrement dans les régions où l'élimination des déchets est déficiente et où la revente de matériel usagé est courante. Une seringue de sécurité autobloquante comporte à la fois un mécanisme qui empêche sa réutilisation et un mécanisme de protection qui protège la canule souillée de tout contact, afin de réduire les risques de piqûre. Selon l'art antérieur, il existe de nombreux dispositifs permettant de répondre à ces impératifs. Toutefois, la plupart de ces dispositifs ne sont pas compatibles avec les moyens de production automatisés de sorte qu'ils ne peuvent être envisagés pour un traitement thérapeutique de masse et qu'ils ont un effet très défavorable sur le coût de la seringue. Cette insuffisance des dispositifs de l'art antérieur provient notamment de l'augmentation du diamètre de la seringue, par la présence du dispositif, rendant ladite seringue incompatible aves les entraxes des machines de remplissage et avec les plaques à cheminées standard. De plus, la nécessité éventuelle d'introduire une partie du mécanisme à l'intérieur de la seringue avant son remplissage ou la complexité d'installation dudit dispositif, laquelle installation nécessitant des combinaisons de mouvements complexes, ou la réalisation d'opérations d'assemblage simultanément aux deux extrémités du corps de seringue, rendent délicate l'utilisation d'automates pour la mise en oeuvre d'une production automatisée.

Le document WO 2006/00742 décrit un dispositif de sécurité autobloquant comprenant un bouclier venant coiffer la canule après l'acte d'injection. Ce document est représentatif des insuffisances de l'art antérieur. Ledit dispositif est monté à l'intérieur du corps de la seringue et utilise à cette fin un corps spécifique épaulé, bouché par un bouchon particulier auquel estfixée la canule. Le mouvement du bouclier est guidé sur

la canule et dans le corps ce qui implique le respect d'une concentricité soignée entre ladite canule et le corps de seringue pour éviter tout risque de coincement ou de flexion de la canule. Selon un premier mode de réalisation de cet art antérieur, la protection de la canule est semi-automatique dans le sens où elle est obtenue en continuant le déplacement du piston après l'injection, ce qui a pour effet de pousser le bouclier le long de la canule jusqu'à une position de verrouillage. Selon un autre mode de réalisation de cet art antérieur, la protection de la canule est automatique et utilise à cette fin un ressort hélicoïdal, guidé également autour de la canule, pour mouvoir le bouclier.

À l'état de livraison de la seringue, le ressort est comprimé, maintenu dans cette configuration par une pince à griffes, coopérant avec une bague à l'intérieur du corps de ladite seringue.

Après la réalisation de l'injection, un aménagement du piston resserre les griffes de la pince, laquelle est alors désolidarisée de la bague, libérant le ressort qui déplace le bouclier jusqu'à ce que celui-ci recouvre l'extrémité de la canule. La nécessité de loger le ressort implique d'allonger la canule de manière conséquente. À l'état de livraison, le ressort est comprimé au maximum de sa compression autorisée, de sorte que cet état est instable et que la mécanisme de couverture du bouclier est susceptible de se déclencher de manière accidentelle, par exemple, si la seringue est soumise à des vibrations, notamment au cours de son transport.

L'invention vise à résoudre les inconvénients de l'art antérieur et concerne à cette fin un dispositif de verrouillage automatique d'une canule d'injection, notamment pour une seringue, lequel dispositif comporte :
a. un bouclier sensiblement tubulaire de longueur égale ou supérieure à celle de la canule à verrouiller ;
b. un moyen de guidage apte à guider ledit bouclier selon une translation axiale parallèle à l'axe du cylindre ;
c. des moyens élastiques, dits de rappel, aptes à exercer une pression axiale sur le bouclier entre une première position, dite proximale ou d'armement, où ledit bouclier découvre la canule, et une deuxième position, dite distale ou de sécurité, où ledit bouclier recouvre axialement la canule ;
d. des moyens de verrouillage, aptes à bloquer le bouclier dans la position distale ;
e. **caractérisé en ce qu'il** comprend des moyens d'arrêt, aptes à arrêter le bouclier dans une position axiale, dite intermédiaire ou pré-opératoire, entre la position distale et la position proximale, les moyens élastiques de rappel exerçant une pression axiale orientée de la position proximale vers la position distale, par l'intermédiaire du bouclier, sur lesdits moyens d'arrêt ; et
f. des moyens pour escamoter les moyens d'arrêt lorsque le bouclier passe de la position proximale à la position distale sous l'effet de la détente des moyens élastiques de rappel.

Ainsi la configuration pré-opératoire, dans laquelle les moyens élastiques n'exercent qu'une faible pression sur le bouclier, est stable, l'armement provoque le bandage des moyens élastiques, l'action se réalisant en même tant que la pénétration de la canule dans le tissu du sujet recevant l'injection. Lesdits moyens élastiques provoquent le verrouillage de manière automatique dès que le bouclier est relâché depuis la position proximale, c'est-à-dire dès que la canule est retirée du tissu du sujet.

Ainsi, ce dispositif est indépendant de tout mécanisme de piston, par conséquent, il ne nécessite pas l'insertion de moyens à l'intérieur du corps de la seringue. Il est également compatible avec d'autres moyens intrusifs tels qu'un cathéter. Le dispositif objet de l'invention est également mécaniquement indépendant de la canule et comporte son propre système de guidage. L'ensemble est autonome et facilement installé sur le système d'injection, selon une seule direction d'assemblage axiale, il est par conséquent aisément mis en production par des moyens d'assemblage automatisés.

L'invention est avantageusement mise en oeuvre selon les modes de réalisation exposés ci-après, lesquels sont à considérer individuellement ou selon toute combinaison techniquement opérante.

Dans tout le texte les termes « lier » et « liaison » désignent une relation fonctionnelle mécanique entre deux éléments supprimant au moins un degré de liberté relatif entre les dits éléments.

Le terme « fixer » désigne la réalisation d'une liaison complète entre deux éléments.

Avantageusement, les moyens de guidage sont tubulaires, le bouclier coulissant sur ou à l'intérieur desdits moyens. Ainsi l'ensemble est facilement installé de manière concentrique sur un corps de seringue cylindrique.

Avantageusement, les moyens élastiques de rappel sont constitués d'un ressort hélicoïdal. Ce mode de réalisation est économique et compatible avec un montage concentrique au corps de seringue du dispositif.

Selon un mode de réalisation particulier, le ressort hélicoïdal est un ressort de traction. Ainsi, la présence dudit ressort est aisément masquée par le bouclier lui-même, et le ressort est moins sujet au coincement par les phénomènes de flambage lorsqu'il est bandé.

Selon un mode de réalisation avantageux du dispositif objet de l'invention, les moyens d'arrêt comportent une rainure, dite principale, s'étendant axialement dans le bouclier et une rainure, dite d'arrêt, s'étendant dans le bouclier et sécante de la rainure principale. Ce mode est simple de réalisation et permet un arrêt particulièrement stable en position intermédiaire du bouclier.

Avantageusement, le bouclier coulisse à l'intérieur des moyens de guidage, dits embase, et les moyens élastiques consistent en un ressort hélicoïdal dont les spires sont contenues à l'intérieur de ladite embase. Ce mode de réalisation permet de protéger de l'environnement extérieur tous les éléments du dispositif par ladite embase.

Avantageusement, les moyens de verrouillage comprennent :
di. une rainure radiale pratiquée dans l'embase ;
dii. des moyens élastiques radialement expansibles selon une flexion autour d'un axe longitudinal, portés par le bouclier et aptes à coopérer avec ladite rainure radiale de l'embase.

Ce mode de réalisation permet de profiter du diamètre de l'embase et du bouclier pour réaliser le verrouillage sur une surface de blocage importante assurant la sûreté de ce verrouillage.

L'invention concerne également une seringue comportant un corps cylindrique et un dispositif de verrouillage selon l'un quelconque des modes de réalisation précédent, dans laquelle l'embase est centrée sur l'extérieur du corps de la seringue et le bouclier coulisse dans un espace radial compris entre le corps de la seringue et l'embase. Ce mode de réalisation permet d'adapter ledit dispositif par simple glissement de celui-ci sur le corps de la seringue.

Avantageusement, la seringue objet de l'invention comporte un ressort hélicoïdal de traction, l'extrémité fixe dudit ressort étant liée au corps de seringue par l'intermédiaire d'une liaison élastique apte à accommoder un défaut de concentricité relatif entre ledit ressort et le corps de la seringue. Ainsi le dispositif est connecté à la seringue sans générer de contraintes d'hyperstatisme.

Avantageusement, la liaison élastique est réalisée par une partie d'un capuchon souple apte à recouvrir la canule, ledit capuchon étant scindable axialement en deux parties. Ainsi le dispositif objet de l'invention est facilement installé, en même temps que la canule protégée sur le corps de la seringue par des moyens automatisés en une seule opération.

Avantageusement, le bouclier est constitué d'un matériau métallique et comporte une lumière pour le mirage de la seringue en configuration pré-opératoire. Ce mode de réalisation permet l'utilisation d'un bouclier particulièrement fin tout en étant résistant sans gêner le contrôle automatisé.

Avantageusement, la longueur du bouclier est 1,5 fois la longueur de la canule. Cette proportion est optimale en matière de course et d'encombrement du ressort.

Avantageusement, le ressort est compris dans un espace radial entre le bouclier et le corps de seringue. Ainsi le ressort n'est pas visible de l'extérieur de la seringue.

Avantageusement, le ressort est constitué d'un matériau superélastique. Ainsi, le ressort utilise un fil de faible diamètre tout en procurant une course importante et n'oppose par une résistance excessive lors de la pénétration de la canule dans le tissu du sujet.

L'invention concerne également un procédé pour le remplissage d'un corps de seringue prêt à remplir **caractérisé en ce qu'il** comprend les étapes consistant à :
i. glisser axialement sur l'extrémité de la seringue comportant la canule, un dispositif selon l'invention en une seule opération ;
ii. placer la seringue ainsi équipée dans une plaque à cheminées ;
iii. remplir ladite seringue.

Ce procédé de fabrication est aisément automatisable, sa mise en oeuvre étant compatible avec les moyens de production de l'art antérieur.

L'invention est exposée ci-après selon ses modes de réalisation préférés, nullement limitatifs, et en référence aux figures 1 à 6, dans lesquelles :
- la figure 1 représente, selon un schéma de principe en vue de face et en coupe longitudinale, un exemple de réalisation du dispositif objet de l'invention, utilisant des moyens élastiques sous la forme d'un ressort hélicoïdal de compression, figure 1A, en configuration pré-opératoire ou intermédiaire, figure 1B, en configuration d'armement et figure 1C en configuration de verrouillage ;
- la figure 2, montre selon une vue de face un exemple de réalisation du dispositif objet de l'invention selon une vue de face en éclaté, utilisant un ressort hélicoïdal de traction, les lignes en pointillé représentant des arêtes cachées ;
- la figure 3 montre selon un vue en perspective en éclaté un exemple d'assemblage du dispositif de la figure 2 sur une seringue ;
- la figure 4 illustre selon une vue en coupe AA définie figure 3, le fonctionnement du dispositif de la figure 2 appliqué à une seringue, figure 4A, en configuration pré-opératoire, figure 4B, en configuration d'armement et figure 4C en configuration de verrouillage ;
- la figure 5 est un diagramme montrant un exemple d'évolution de la force de rappel d'un ressort métallique superélastique ;
- et la figure 6 est un logigramme du procédé objet de l'invention.

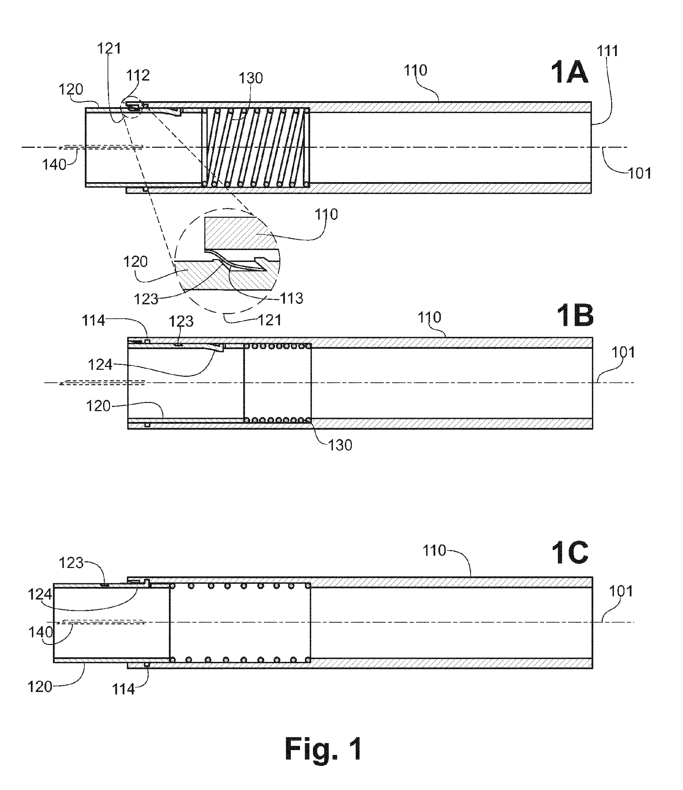

Figure 1, selon un exemple de réalisation, le dispositif objet de l'invention est constitué d'éléments essentiellement tubulaires s'étendant selon un axe (101) dit longitudinal. Selon cet exemple de réalisation, un bouclier (120) apte à couvrir une canule (140), coulisse à l'intérieur d'une embase (110). Cette embase s'étend longitudinalement, entre une extrémité (111), dite proximale, et une extrémité (112), dite distale. Ladite canule (140) fait partie d'une seringue d'injection ou d'un cathéter (non représentés), le dispositif objet de l'invention étant monté sur ceux-ci de sorte que l'extrémité (112) distale de l'embase (110) se trouve du côté de ladite canule (140). Des moyens élastiques (130), ici sous la forme d'un ressort hélicoïdal de compression, exercent une pression axiale, selon l'axe (101) longitudinal, sur ledit bouclier (120).

Figure 1A, le bouclier (120) se trouve dans une position, dite intermédiaire. Dans cette position du bouclier, la canule (140) est partiellement découverte. Le bouclier est maintenu dans cette position par le ressort (130) qui exerce sur ledit bouclier (120) une force axiale orientée de l'extrémité (111) proximale de l'embase vers l'extrémité (112) distale de ladite embase (110), le bouclier étant arrêté axialement par des moyens (121) d'arrêt. Selon une vue de détail, lesdits moyens (121) d'arrêt comportent, selon u exemple de réalisation, une lame (113) élastique fixée à l'extrémité (112) distale de l'embase, laquelle lame coopère avec une encoche (123) ou une rainure pratiquée dans le bouclier (120). Lorsque, la lame étant dans l'encoche, le bouclier (120) se déplace de l"extrémité (112) distale de l'embase vers l'extrémité (111) proximale de ladite embase, la forme de la rainure (123) ou de l'encoche escamote ladite lame (113).

Lorsque, la lame (113) étant repliée le long de la paroi de l'embase (110), le bouclier se déplace de l'extrémité (111) proximale de l'embase vers l'extrémité (112) distale de ladite embase, la forme de la rainure (123) ou encoche fait que ladite lame ne peut pas pénétrer dans ladite rainure (123) au passage du bouclier.

Ainsi, figure 1B, lorsque le bouclier (120) est poussé vers l'extrémité (111) proximale de l'embase, celui-ci comprime le ressort (130) et découvre axialement la canule (140). Rien ne maintien le bouclier (120) dans cette position axiale dite d'armement, si ce n'est le sujet dans lequel est réalisée l'injection.

Ainsi, figure 1C, si depuis la position d'armement de la figure 1B, le bouclier est relâché, le ressort (130) pousse ledit bouclier (120) jusqu'à ce que celui-si soit arrêté par les moyens de verrouillages. Selon un exemple de réalisation du dispositif objet de l'invention, lesdits moyens de verrouillage comprennent une griffe (124) élastique apte à fléchir selon une direction radiale et faisant saillie par rapport au fût du bouclier lorsqu'elle n'est pas contrainte. Ladite griffe (124) coopère avec une rainure (114) à l'intérieur de l'embase (110). En position verrouillée, le bouclier recouvre entièrement la canule (140).

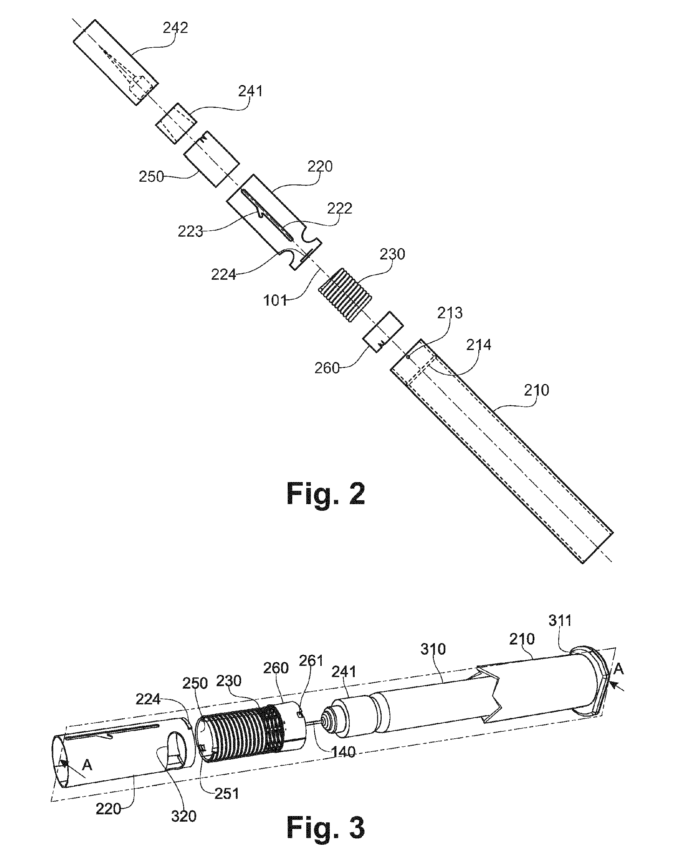

Figure 2 selon un autre exemple de réalisation, le dispositif objet de l'invention comporte une embase (210) tubulaire, un bouclier (220) et des moyens (230) élastiques sous la forme d'un ressort hélicoïdal de traction. Les moyens d'arrêt consistent en deux rainures (222, 223) pratiquées sur le bouclier (220) lesquelles rainures coopèrent avec un pion (213) fixé à l'extrémité distale de l'embase (210) et faisant saillie à l'intérieur de ladite embase (210). Les moyens de verrouillage consistent, selon cet exemple de réalisation, en une rainure (214) pratiquée à l'intérieur de l'embase, qui coopère avec des pattes annulaires (224) faisant saillie radialement par rapport au fût du bouclier (220) et déformables élastiquement selon un flexion autour d'une axe parallèle à l'axe longitudinal (101). Le ressort (230) est fixé à deux bagues (250, 260), une bague fixe (250), laquelle, selon un exemple de réalisation est fixé à un point axialement fixe par l'intermédiaire d'une liaison radialement élastique au moyen d'une rondelle (241) constituée d'un élastomère. Selon cet exemple de réalisation, le dispositif objet de l'invention comprend également un bouchon (242) souple apte à couvrir une canule. Selon cet exemple de réalisation du dispositif objet de l'invention, le ressort (230) de rappel est fixé aux bagues (250, 260) par l'intermédiaire de pattes de fixatio découpées et pliées à l'une des extrémités desdites bagues en engageant une des spires dudit ressort dans lesdites pattes qui agissent comme des pinces.

Figure 3, le dispositif objet de l'invention est aisément adapté à une seringue (310). La rondelle (241) élastiques est fixée par coincement à l'extrémité de la seringue côté canule (140), la bague (250) fixe étant fixée, par exemple par collage, à ladite rondelle (241) radialement élastique. Le ressort (230) est fixé à une de ses extrémités à la bague (250) fixe, par l'intermédiaire des pattes (251) de fixation, et à son autre extrémité, à la bague (260) mobile par l'intermédiaire des pattes (261) de fixation de cette bague. Le diamètre extérieur de la bague (250) fixe est inférieur au diamètre intérieur du ressort, et le diamètre extérieur du ressort (230) est inférieur au diamètre intérieur du bouclier (220) et de la bague (260) mobile. Le diamètre extérieur du bouclier (220) et de la bague (260) mobile est sensiblement inférieur au diamètre intérieur de l'embase. Le diamètre extérieur de la bague (260) mobile est sensiblement équivalent au diamètre extérieur du bouclier (220) de sorte que lorsque le bouclier coulisse vers la prise digitale (311) de la seringue (310), il entraîne avec lui la bague (260) mobile, provoquant une tension du ressort. Ainsi le ressort exerce une tension sur la bague mobile qui tend à pousser le bouclier (220) vers la canule (140) de la seringue. Cette tension du ressort est exercée pour toute position du bouclier (220) entre la position d'armement et la position de verrouillage.

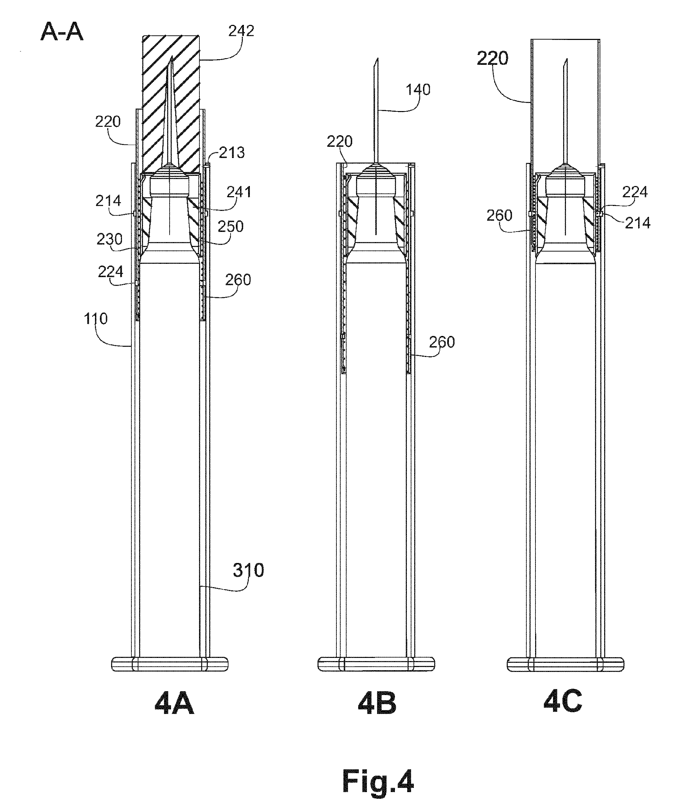

Figure 4A, en position de pré-opératoire, qui correspond à l'état de livraison d'une seringue pourvue d'un dispositif conforme à l'invention, le bouclier (220) recouvre axialement la canule (140) de manière partielle. La canule est protégée et fermée par un capuchon (252) qui la protège de toute souillure et de tout contact. Dans cette configuration la seringue est apte à être remplie sur des moyens de production automatisés. Avantageusement, l'embase (210) est constituée d'une matière transparente de sorte qu'elle n'obstrue pas la visibilité du contenu de ladite seringue.

Selon un exemple de réalisation avantageux, le diamètre extérieur de l'embase (210) est du diamètre standardisé immédiatement supérieur au diamètre extérieur du corps de seringue auquel le dispositif objet de l'invention est adapté. Ainsi, à titre d'exemple non limitatif, pour une seringue pré-remplie contenant 2 ml d'héparine sodique, dont le corps est d'un diamètre standardisé de 6,84 mm, le diamètre extérieur de l'embase est pris au plus égal à 8,96 mm. Ainsi, le remplissage d'une pluralité de seringues de ce type est réalisé avec une plaque à cheminées et un réglage d'entraxe des canules de la machine de remplissage automatique correspondant à ceux des seringue de diamètre immédiatement supérieur.

Dans cette position pré-opératoire, le ressort pousse le bouclier (220) contre le pion (213) inséré à l'extrémité distale de l'embase. Ledit pion est alors logé dans la rainure (223 figure 2) d'arrêt, sécante à la rainure (222 figure 2) principale du bouclier. L'ensemble du dispositif objet de l'invention est maintenu axialement sur la seringue par la rondelle (241) radialement élastique à laquelle est fixée la bague (250) fixe. L'élasticité radiale de cette rondelle (250) permet d'absorber les légers défauts de centrage entre le corps de la seringue, la canule et l'extrémité de ladite seringue sans risque de coincer le dispositif objet de l'invention.

Figure 4B, pour réaliser une injection, l'utilisateur retire le capuchon (242) ce qui a pour effet de découvrir l'extrémité de la canule (140). En piquant le sujet et en y faisant pénétrer la canule (140) le pion (213) quitte la rainure d'arrêt et entre dans la rainure principale du bouclier (220), ledit bouclier réalisant une légère rotation autour de l'axe longitudinal. Le bouclier recule vers l'extrémité proximale de l'embase en tendant le ressort (230). Tant dans la position pré-opératoire qu'au cours de ce recul, du bouclier, les pattes (224) annulaires du bouclier sont en contact avec l'intérieur de l'embase (210). La position extrême d'armement est atteinte lorsque le sommet du bouclier (220) affleure l'extrémité distale de l'embase (210).

Figure 4C, l'injection étant réalisée, la canule étant extraite progressivement du sujet, le ressort (230) tire la bague (260) mobile qui pousse le bouclier. Au cours de cette translation axiale du bouclier, le pion (213) reste dans la rainure principale de sorte que ladite translation se poursuit jusqu'à ce que les pattes (224) annulaires pénètrent dans la rainure (214) annulaire de l'embase (210). Cette introduction des pattes annulaire dans ladite rainure est provoquées par l'élasticité desdites pattes (224) qui, en l'absence de sollicitation radiale, font saillie par rapport au fût du bouclier (220).

Dans cette position le bouclier est verrouillé en translation et recouvre axialement la canule (140)..

Afin de réduire le diamètre du dispositif objet de l'invention, le ressort est avantageusement constitué d'un matériau superélastique, par exemple un fil d'alliage comprenant du nickel (Ni) et du titane (Ti) à l'état austénitique et ayant subit un traitement thermo-mécanique apte à favoriser l'induction d'une transformation martensitique réversible de ladite austénite par déformation. Ainsi, ledit ressort permet de produire un allongement important avec un diamètre de fil réduit. De plus, l'induction de martensite par déformation se réalise sous une contrainte quasi constante ou faiblement évolutive, que qui permet de disposer d'un ressort de faible raideur pendant les premiers contacts du bouclier avec le sujet, au cours de la pénétration de la canule.

Avantageusement, la longueur axiale du bouclier est 1,5 fois la longueur axiale en saillie de la canule. Ainsi, en configuration pré-opératoire, telle que représenté figure 4A, le bouclier recouvre la moitié de la canule et le ressort est bandé à environ la moitié de son allongement maximum. En configuration d'armement, figure 4B, le bouclier découvre complètement la canule.

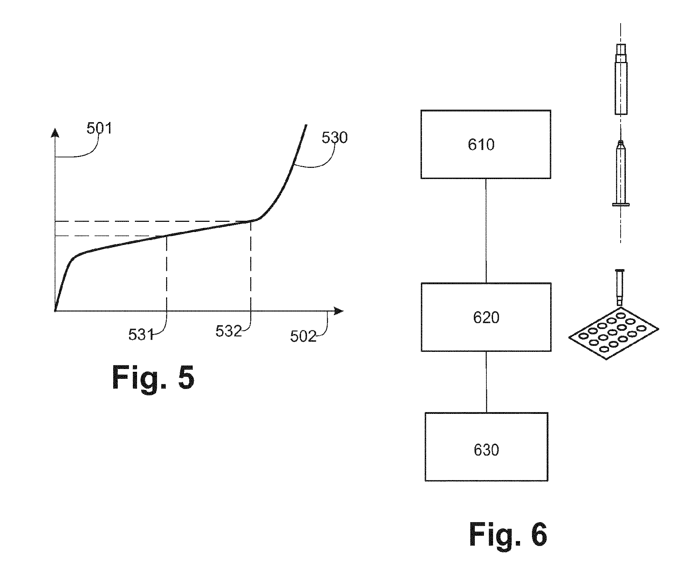

Figure 5, l'évolution (530) de la force (502) en fonction de l'allongement (502) du ressort lorsque ledit ressort est un matériau au comportement superélastique, présente un palier dans lequel la force de rappel est sensiblement constante avec une raideur faible dans cette partie de l'évolution. Ce palier correspond à l'induction de matensite sous l'effet de la déformation. Ainsi, avantageusement, les caractéristiques du ressort sont choisies de telle sorte qu'en configuration pré-opératoire (531) ledit ressort se trouve sensiblement au milieu du palier d'induction martensitique. Ainsi, lors de passage de la configuration pré-opératoire (531) à la configuration d'armement (532) la force de rappel opposée par ledit ressort est sensiblement constante. L'arrivée en butée du pion dans la rainure du bouclier évite que le bouclier ne soit repoussé au delà de la position d'armement. Selon un mode de réalisation particulier, les caractéristique du ressort sont fixées de sorte que lorsque le bouclier s'approche de la position d'armement, la force (530) de rappel augmente rapidement dissuadant l'utilisateur de poursuivre en ce sens.

En revenant à la figure 3, pour des raisons de résistance, le bouclier est avantageusement constitué d'un matériau métallique, par exemple un alliage de titane pour conférer audit bouclier résistance et légèreté. Afin de permettre le contrôle de la qualité du fluide contenu dans la seringue, notamment par mirage, ledit bouclier comporte avantageusement des lumières (320) pour réaliser ce contrôle.

Figure 6, selon un exemple de réalisation le procédé objet de l'invention comporte une étape (610) consistant à installer le dispositif objet de l'invention sur la seringue. Ce dispositif selon le mode de réalisation de la figure 4, est enfilé sur la seringue en une seule opération axiale, facilement automatisable. Au cours d'une étape (620) ultérieure, l'ensemble ainsi constitué est placé dans ne plaque à cheminées, de sorte à être rempli au cours d'une étape (630) de remplissage sur des moyens de remplissage automatisés.

La description ci-avant et les exemples de réalisation montrent que l'invention atteint les objectifs visés, en particulier, le faible accroissement de diamètre consécutif à l'installation du dispositif objet de l'invention sur la seringue conserve la compatibilité de celle-ci avec les moyens de production automatisés. L'ensemble du dispositif est facilement assemblé en dehors de a seringue et est ensuite glissé sur ladite seringue en un seul mouvement axial, facilement réalisable par un automate.

## Revendications

1. Dispositif de verrouillage automatique d'une canule (140) d'injection, notamment pour une seringue, lequel dispositif comporte :
a. un bouclier (120, 220) sensiblement tubulaire de longueur égale ou supérieur à celle de la canule à verrouiller ;
b. un moyen (110, 210) de guidage apte à guider ledit bouclier (120) selon une translation axiale parallèle à l'axe (101) du cylindre ;
c. des moyens (130, 230) élastiques, dits de rappel, aptes à exercer une pression axiale sur le bouclier (120, 220) entre une première position, dite proximale ou d'armement, où ledit bouclier (120, 220) découvre la canule (140) et une deuxième position, dite distale ou de sécurité, où ledit bouclier (120, 220) recouvre axialement la canule ;
d. des moyens (124, 114,224, 214) de verrouillage, aptes à bloquer le bouclier dans la position distale ;
e. **caractérisé en ce qu'il** comprend des moyens (121, 123, 113, 223, 213) d'arrêt aptes à arrêter le bouclier (120, 220) dans une position axiale, dite intermédiaire ou pré-opératoire, entre la position distale et la position proximale, les moyens (130, 230) élastiques de rappel exerçant une pression axiale orientée de la position proximale vers la position distale, par l'intermédiaire du bouclier (120, 220), sur lesdits moyens d'arrêt; et
f. des moyens (123, 222) pour escamoter les moyens d'arrêt lorsque le bouclier passe de la position proximale à la position distale sous l'effet de la détente des moyens élastiques (130,; 230)de rappel.

2. Dispositif selon la revendication 1, dans lequel les moyens (110, 210) de guidage sont tubulaires, le bouclier coulissant sur ou à l'intérieur desdits moyens.

3. Dispositif selon la revendication 1, dans lequel les moyens élastiques (130, 230) de rappel sont constitués d'un ressort hélicoïdal.

4. Dispositif selon la revendication 3, dans lequel le ressort (230) hélicoïdal est un ressort de traction.

5. Dispositif selon la revendication 1, dans lequel les moyens d'arrêt comportent une rainure (222), dite principale, s'étendant axialement dans le bouclier (220) et une rainure (223), dite d'arrêt, s'étendant dans le bouclier et sécante de la rainure principale.

6. Dispositif selon les revendications 2, dans lequel le bouclier (220, 120) coulisse à l'intérieur des moyens (110, 210) de guidage, dits embase, et que les moyens (130, 230) élastiques de rappel consistent en un ressort hélicoïdal dont les spires sont contenues à l'intérieur de ladite embase (110,210).

7. Dispositif selon la revendication 6, dans lequel les moyens de verrouillage comprennent :
di. une rainure (214) radiale pratiquée dans l'embase ;
dii. des moyens (224) élastiques radialement expansibles selon une flexion autour d'un axe longitudinal, portés par le bouclier et aptes à coopérer avec ladite rainure radiale de l'embase.

8. Seringue (310), notamment prête à remplir, comportant un corps cylindrique et un dispositif selon la revendication 6, **caractérisée en ce que** l'embase (210) est centrée sur l'extérieur du corps de la seringue et que le bouclier (220) coulisse dans un espace radial compris entre le corps de la seringue et l'embase (210).

9. Seringue selon la revendication 8, comportant un ressort (230) hélicoïdal de traction dans laquelle l'extrémité fixe dudit ressort est liée au corps de seringue par l'intermédiaire d'une liaison (241) élastique apte à accommoder un défaut de concentricité relatif entre ledit ressort et le corps de la seringue.

10. Seringue selon la revendication 9, dans laquelle la liaison élastique est réalisée par une partie d'un capuchon souple apte à recouvrir la canule, ledit capuchon étant scindable axialement en deux parties (241, 242).

11. Seringue selon la revendication 8, dans laquelle le bouclier (220) est constitué d'un matériau métallique et qu'il comporte une lumière (320) pour le mirage de ladite seringue en configuration pré-opératoire.

12. Seringue selon a revendication 8, dans laquelle la longueur du bouclier (220) est 1,5 fois la longueur de la canule (140).

13. Seringue selon la revendication 9, dans laquelle le ressort (230) est compris dans un espace radial entre le bouclier (220) et le corps de seringue.

14. Seringue selon la revendication 13, dans laquelle le ressort (230) est constitué d'un matériau superélastique.

15. Procédé pour le remplissage d'un corps de seringue prêt à remplir **caractérisé en ce qu'il** comprend les étapes consistant à :
i. glisser (610) axialement sur l'extrémité de la seringue comportant la canule, un dispositif selon la revendication 7 en une seule opération ;
ii. placer (620) la seringue ainsi équipée dans une plaque à cheminées ;
iii. remplir (630) ladite seringue.
